# EUROPEAN PATENT APPLICATION

(11) **EP 1 214 955 A1**
(43) Date of publication of application: **19.06.2002**
(21) Application number: 00830826.4
(22) Date of filing: 15.12.2000
(51) Int. Cl.: A61M 5/165, B01D 63/08, B01D 61/18

(54) **Flat filter for infusion, transfusion and the like medical lines**

(71) Applicant: Industrie Borla SpA, 10024 Moncalieri (Torino) (IT)
(72) Inventor: Guala, Ernesto, 10133 Torino (IT); Guala, Gianni, 10133 Torino (IT)
(74) Representative: Buzzi, Franco

(57) **Abstract**

A flat filter (1) for infusion, transfusion and the like medical lines, comprising a first plate (2) and a second plate (3) hermetically coupled to each other and a filtering membrane (14) interposed therebetween. A first plate (2) has an inlet (8) for a liquid to be filtered and an outlet (9) for the liquid filtered through the filtering membrane (14). The inlet (8) and the outlet (9) are arranged in close proximity to each other and are formed in a single common hollow projection (4) integrally protruding from the first plate (2) near to one end (15) thereof.

## Description

The present invention is generally related to filtering devices for infusion, transfusion and the like medical lines.

More particularly the invention is directed to a flat filter for such medical lines, comprising a first plate and a second plate hermetically coupled to each other in a superimposed condition and between which a filtering membrane is interposed, wherein the first plate has an inlet for the liquid to be filtered and an outlet for the liquid filtered through said membrane.

Traditionally, in the known flat filters of the above-referenced type the inlet and the outlet are provided as two separate tubular connectors more or less spaced-apart from each other along the longitudinal direction of the first plate.

This arrangement involves practical problems for manufacturing the filter, which is normally made of moulded plastic material, mainly related to the relative complexity of the mould designed to manufacture the first plate with the mutually spaced-apart inlet and outlet connectors.

Moreover, with the above construction the known filters have a remarkable size, particularly in the longitudinal direction, which involves encumbrance problems in use.

The object of the present invention is to overcome the above drawbacks, and to provide a flat filter of the type set forth at the beginning having, with the same surface extension of the filtering membrane, a reduced encumbrance size with respect to the similar conventional filters, and which moreover can be manufactured in a more simple and economical way while being more comfortable to the patient when in use.

According to the invention this object is achieved essentially by the fact that the inlet for the liquid to be filtered and the outlet for the filtered liquid are arranged in close proximity to each other and are formed in a single common hollow projection integrally protruding from the first plate near to one end thereof.

By virtue of this idea of solution firstly manufacturing of the filter is appreciably simplified, since moulding of the first plate is made easy. Secondly the mutual close proximity of the liquid inlet and outlet enables reducing the longitudinal dimension of the filter, which can thus be made, with the same filtering surface of the membrane, at a lower cost and with the additional advantage of a more comfortable and convenient use by the patient undergoing and infusion or transfusion treatment.

The invention will now be disclosed in detail with reference to the accompanying drawings, purely provided by way of non limiting example, in which:

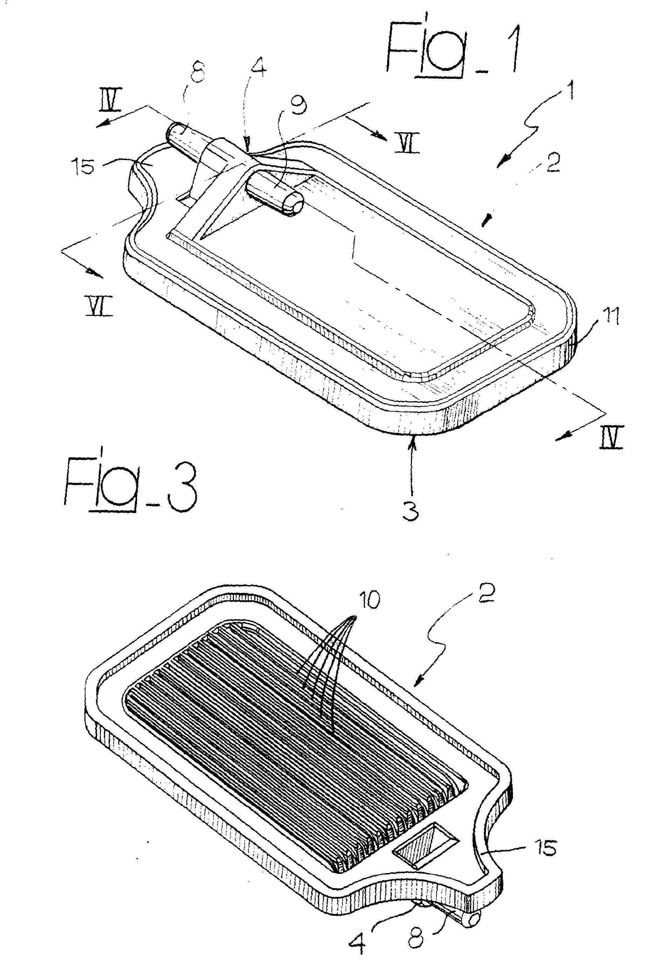

- Figure 1 is a diagrammatic perspective view of a flat filter for medical lines according to the invention,

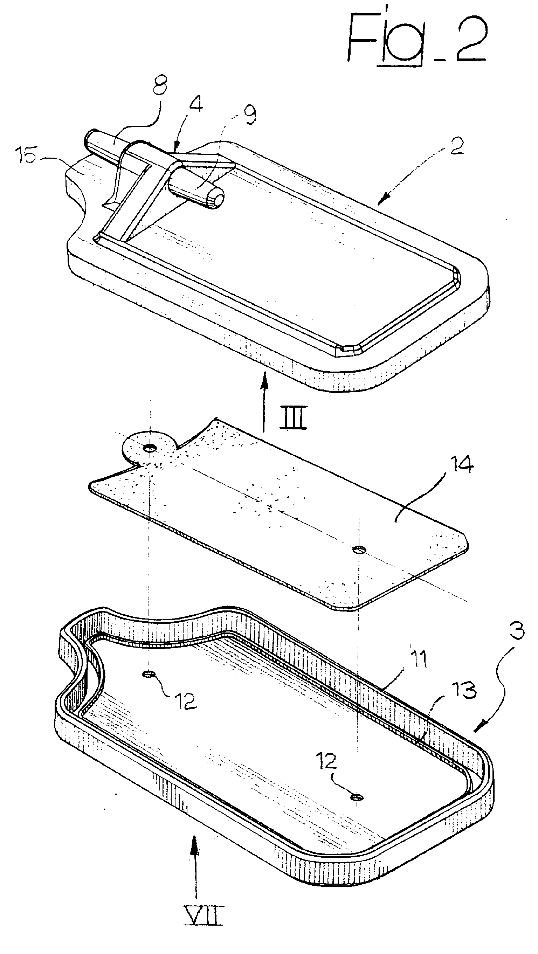

- Figure 2 is an exploded view of Figure 1,
- Figure 3 is a perspective view from below along arrow III of Figure 2,
- Figure 4 is a longitudinally sectioned view along line IV-IV of Figure 1,
- Figure 5 is an exploded view of Figure 4,
- Figure 6 is a transversally sectioned view along line VI-VI of Figure 1, and
- Figure 7 is a perspective view from below along line VII of Figure 2.

Referring to the drawings, reference numeral 1 generally designates a flat filter according to the invention designed to be fitted into an infusion, transfusion or the like medical line.

The filter 1, which is entirely made by moulding of a bio-compatible and normally transparent plastic material, comprises a first plate 2 and second plate 3 hermetically coupled to each other (for instance by ultra-sound welding, adhesive bonding or equivalent system) in a superimposed condition.

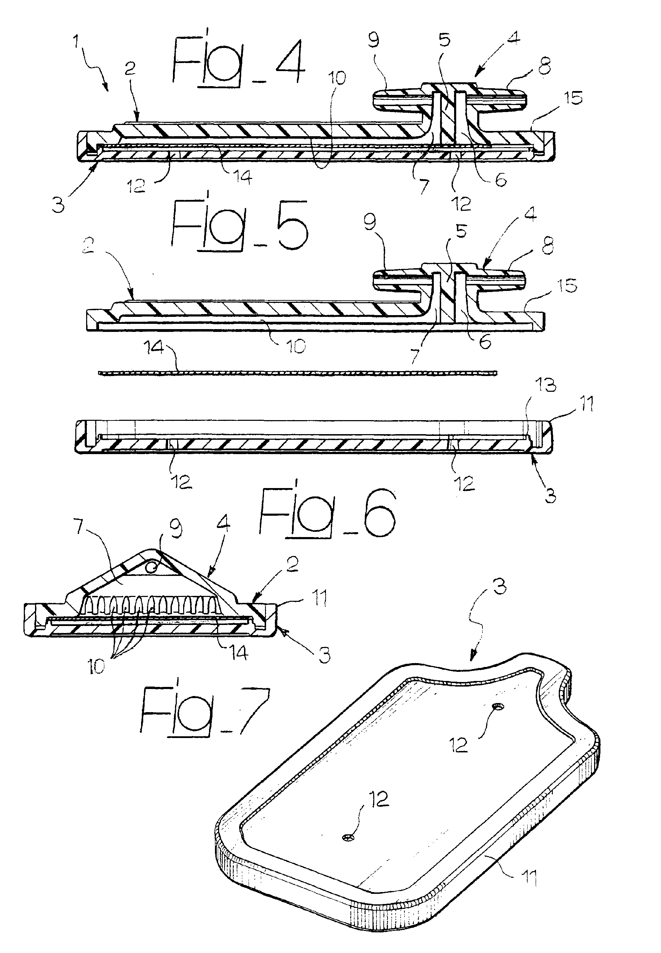

The first plate 1 has on its outer face a hollow projection 4 integrally formed upon moulding near to a tapered end 15 of the plate 1. As better shown in Figures 4 trough 6, the hollow projections 4 has a substantially triangular design and is divided by an integral central wall 5 into a first and a second manifolds 6, 7 each of which is centrally communicating, in correspondence of the top of the hollow projection 4, with respective juxtaposed tubular connectors 8, 9. The tubular connector 8 defines an inlet for a liquid to be filtered, and the tubular connector 9 defines an outlet for the filtered liquid. The inlet and outlet connectors 8, 9 are intended to be connected, in use, with the ends of respective flexible tubings of the medical line.

The inner wall of the first plate 2 is integrally formed with a number of equally-spaced longitudinal ribs 10, whose function shall be clarified in the following.

The second plate 3 has a tray construction, with a raised perimetral edge 11 within which the perimetral edge of the first plate 2 is fitted and hermetically fixed, such as previously explained.

The second plate 3 is additionally formed on its bottom with a pair of through holes 12 into which respective vent valves (not shown in the drawings) are intended to be fitted for bleeding out the air contained within the liquid during filtration thereof.

The bottom of the second plate 3 has, at a distance from its perimetral edge 11, an annular rib 13 acting as a support for the perimetral edge of a filtering membrane 14.

As clearly shown in figures 4 and 6, the membrane 14 is hermetically clamped between the plates 2 and 3 and is facing towards the longitudinal ribs 10 of the plate 2, which define between one another flow paths for the filtered liquid coming from the inlet 8 and directed towards the outlet 9 of the filter 1.

Naturally, the details of construction and the embodiments may be widely varied with respect to what has been disclosed and illustrated, without thereby departing from the scope of the present invention such as defined in the attended claims.

## Claims

1. Flat filter (1) for infusion, transfusion and the like medical lines, comprising a first plate (2) and a second plate (3) hermetically coupled to each other in a superimposed condition and between which a filtering membrane (14) is interposed, wherein the first plate (2) has an inlet (8) for a liquid to be filtered and an outlet (9) for the liquid filtered through said filtering membrane (14), **characterized in that** said inlet (8) and said outlet (9) are arranged in close proximity to each other and are formed in a single common hollow projection (4) integrally protruding from said first plate (2) near to one end (15) thereof.

2. Filter according to claim 1, **characterized in that** said hollow projection (4) is formed by a body divided by a central wall (5) into a first and a second manifolds (6, 7) centrally communicating with respective juxtaposed tubular connectors (8, 9) defining said inlet and said outlet, respectively.

3. Filter according to claim 2, **characterized in that** said hollow projection (4) has a substantially triangular design.
